# EUROPEAN PATENT APPLICATION

(11) **EP 2 341 079 A1**
(43) Date of publication of application: **06.07.2011**
(21) Application number: 09290991.0
(22) Date of filing: 23.12.2009
(51) Int. Cl.: C07K 16/42, C07K 14/195

(54) **Immunoglobulin-binding proteins derived from Sac7d and uses thereof**

(71) Applicant: Institut Pasteur, 75724 Paris Cedex 15 (FR); Centre National de la Recherche Scientifique, 75016 Paris (FR)
(72) Inventor: Pecorari, Frédéric, 44880 Sautron (FR); Béhar, Ghislaine, 44580 Bourgneuf en Retz (FR); Colinet, Stéphane, 44000 Nantes (FR)
(74) Representative: Marcadé, Véronique

(57) **Abstract**

The present invention pertains to the field of molecular tools for purifying or detecting human antibodies. More precisely, the invention provides new antibody-binding proteins derived from Sac7d and having the sequence SVKVKF**LLNG**EEKEVDTSKI**RD**V**C**R**Q**GK**N**V**K**F**L**Y**N**DNGK**Y**G**A**G**N**V**D**EKDAPKELL DMLARAEREKK or a variant thereof. These proteins have a high affinity for human IgG, have advantageous stability properties, and do not or only weakly recognize non-human immunoglobulins. The present invention also concerns uses of said proteins, in particular for detection or capture applications.

## Description

The present invention pertains to the field of molecular tools for purifying or detecting human antibodies. More precisely, the invention provides new antibody-binding proteins with high affinities for human IgG, which have advantageous stability properties, and which do not or only weakly recognize non-human immunoglobulins.

Proteins are now often fused to a polypeptide tag such as the hexahistidine tag to facilitate their purification by affinity chromatography (Terpe, 2003). This approach is problematic when the protein of interest cannot be modified easily, like for monoclonal antibodies. For these cases, it is necessary to either find natural reagents or to develop new ones by molecular evolution or chemistry, with specificity and affinity for the protein of interest suitable for affinity chromatography.

Several bacterial surface proteins have been identified as affinity reagents that are commonly used to purify antibodies or some of their fragments. Binding specificities of these proteins differ between source species and antibody subclasses. For instance, Protein G and Protein A from group G *Streptococci* and from *Staphylococcus aureus,* respectively, are able to bind IgG mainly via their Fc region (Bjorck, 1988; Bjorck and Kronvall, 1984; Forsgren and Sjoquist, 1966), while Protein L from *Peptostreptococcus magnus* recognize antibodies through light chain interactions (Bjorck, 1988). Protein A binds strongly human IgG1, IgG2 and IgG4 while Protein G binds strongly all human subclasses. Protein A and G bind strongly rabbit IgG while Protein L binds them weakly (see (Roque et al., 2007) for a review). Thus, depending on the antibody to purify, the choice of the binder is critical. A major drawback of these natural antibody binders is that their recognition spectrum may not fit specific needs.

These limitations make the development of new binders with selectivity different from those of natural binders interesting for antibody purification and detection. For instance, chemical and peptidic mimetics of protein A (D'Agostino et al., 2008; Li et al., 1998) have been designed to obtain binders with broad specificity (see (Fassina et al., 2001; Huse et al., 2002) for references). In this strategy, obtaining a binder with a well defined and desirable selectivity is not a trivial problem. Another approach is to modify selectivity of existing proteins by combinatorial protein design. This was for example used to convert an IgG binding domain from Protein A into an IgA binder (Ronnmark et al., 2002).

The present invention provides alternative reagents mainly specific of human IgG (hIgG). These were obtained by converting the specificity of the Sac7d protein from DNA to hIgG specificity. For doing so, the inventors have used a previously described combinatorial library of the protein Sac7d with randomized potential interaction surfaces (Mouratou et al., 2007) and have selected those that bind to hIgG by ribosome display. Derivatives with nanomolar affinities for hIgG (herafter called "binders" and previously called "Afftins" (Krehenbrink et al., 2008)) were analyzed for their binding specificity, their stability at different pH and their ability to be functionally immobilized on agarose beads. The biophysical properties observed conjugated to a recognition spectrum different from those of Protein A, Protein G and Protein L, make these binders an alternative and advantageous selective reagent for antibody detection and antibody purification by affinity chromatography.

A first aspect of the present invention is hence an immunoglobulin-binding protein of sequence SVKVKFX₁X₂X₃GEEKEVDTSKIRDVCRQGKNVKFLYNDNGKYGAGX₄VX₅EKDAP KELLDMLARAEREKK (SEQ ID No: 1), wherein X₁ to X₅ are, independently from each other, any amino acids, wherein said immunoglobulin-binding protein has a high affinity for human IgG. More specifically, an immunoglobulin-binding protein according to the present invention has an apparent affinity for human IgG defined by Kd ≤ 100 µM. These immunoglobulin-binding proteins are hence suitable for purifying human IgG by affinity chromatography (Graves and Wu, 1974).

The inventors have also observed that variants from SEQ ID No: 1, in which a number of punctual mutations have spontaneously appeared or have been voluntarily obtained in non-randomized positions, can also bind human IgG with a high affinity. Accordingly, the present invention also pertains to an immunoglobulin-binding protein which has an apparent affinity for human IgG so that Kd ≤ 100 µM, and the sequence of which is at least 65%, preferably 70%, more preferably 75%, even more preferably at least 80%, still more preferably at least 90% to 95% identical to that of the C3 binder (SEQ ID No: 2). In particular, the present invention pertains to an immunoglobulin-binding protein whose sequence is derived from SEQ ID No: 1 by 1 to 15, more particularly one, two, three, four, five or six mutations or deletions in positions selected in the group consisting of positions 1-6, 11-20, 23, 25, 27-28, 30, 32, 36-39, 41, 43, 45 and 47-66, wherein said immunoglobulin-binding protein has an apparent affinity for human IgG so that Kd ≤ 100 µM. Particular examples of such variants, illustrated in the examples below, are those in which said one, two, three or four mutations or deletions are in positions selected in the group consisting of positions 13, 24, 36, 38, 47, 50 and 56 (mutations in positions 13, 36, 38, 47, 50 and 56 have been obtained by ribosome display, and position 24 was voluntarily mutated to avoid S-S bridging).

In a preferred embodiment of the present invention, the immunoglobulin-binding protein has an apparent affinity for human IgG superior to that indicated above. For example, the apparent affinity is so that Kd ≤ 10 µM, more preferably Kd ≤ 1 µM, and even more preferably Kd ≤ 500 nM. In the experimental results illustrated below, the obtained binders have apparent affinities for human IgG with Kd raging from 34 to 350 nM. For determining the affinity of an immunoglobulin-binding protein for human IgG, any technique known by the skilled artisan can be used, such as, for example, the technique described in the examples below. The human IgG used for determining the affinity of an immunoglobulin-binding protein according to the invention can be, for example, a pool of IgG from human serum, such as the "Immunoglobulin G from human serum" sold by Fluka A. G. (Buchs, Switzerland) under the reference 56834.

The inventors have also observed that the immunoglobulin-binding proteins according to the present invention do not bind all IgG isotypes with the same strength. Indeed, it appears that these binders bind to IgG with decreasing strength in the following order: hIgG1 > hIgG4 >> hIgG2 > hIgG3. In a preferred embodiment, the immunoglobulin-binding protein has an apparent affinity for human IgG1 so that Kd ≤ 100 µM, preferably Kd ≤ 10 µM, more preferably Kd ≤ 1 µM, and even more preferably Kd ≤ 500 nM. Binders according to the invention with an apparent affinity for human IgG1 so that Kd ≤ 50 nM can be obtained as described in the experimental part below, and are an especially preferred embodiment of the present invention.

A very interesting property of the immunoglobulin-binding proteins obtained by the inventors is their species selectivity. Indeed, the binding of one of the obtained binders to IgG from other species has been investigated with IgG from mouse, rat, sheep, goat, rabbit and pig, and only a weak interaction could be detected for pig. Hence, according to a preferred embodiment of an immunoglobulin-binding protein according to the present invention, said immunoglobulin-binding protein binds selectively to human IgG, i.e., with an affinity at least 2 times, preferably at least 5 times, and more preferably at least 10 times superior to its affinity for IgG from other species. Due to this specificity, immunoglobulin-binding proteins according to the invention can be used instead of anti-hIgG primary or secondary antibodies in immune-assays such as ELISAs, Western blots, *etc.*

According to preferred embodiments of the present invention, illustrated in the examples below, X₁X₂X₃ is selected in the group consisting of LLN, KYK, HSH, LCH, RYL and ARH. Simultaneously or independently, preferred immunoglobulin-binding proteins are so that X₄ is N or K, and/or X₅ is D, N or R. Particular examples of immunoglobulin-binding proteins according to the invention are:

SVKVKFLLNGEEKEVDTSKIRDVCRQGKNVKFLYNDNGKYGAGNVDEKDAPKEL
LDMLARAEREKK (SEQ ID No: 2),
SVKVKFLLNGEEKEVDTSKIRDVCRQGKNVKFLYNDNGKYGAGKVNEKDAPKEL
LDMLARAEREKK (SEQ ID No: 3),
SVKVKFHSHGEEKEVDTSKIRDVCRQGKNVKFLYNDNGKYGAGNVDEKDAPKEL
LDMLARAEREKK (SEQ ID No: 4),
SVKVKFLCHGEEKEVDTSKIRDVCRQGKNVKFLYNDNGKYGAGNVDEKDAPKEL
LDMLARAEREKK (SEQ ID No: 5),
SVKVKFRYLGEEKEVDTSKIRDVCRQGKNVKFLYNDNGKYGAGNVDEKDAPKEL
LDMLARAEREKK (SEQ ID No: 6),

SVKVKFARHGEEKEVDTSKIRDVCRQGKNVKFLYNDNGKYGAGNVREKDAPKEL
LDMLARAEREKK (SEQ ID No: 7),
SVKVKFLLNGEEKEVDTSKIRDVCRQGKNVKFLYNDNGKYGAGNVDEKDVPKEL
LDMLARAEREKK (SEQ ID No: 8),
SVKVKFLLNGEEQEVDTSKIRDVCRQGKNVKFLYNDNGKYGAGNVDEKDAPKEL
LDMLARAEREKK (SEQ ID No: 9)
SVKVKFLLNGEEKEVDTSKIRDVCRQGKNVKFLYNDNGKYGAGNVDKKDAPKEL
LDMLARAEREKK (SEQ ID No: 10)
SVKVKFLLNGEEKEVDTSKIRDVCRQGKNVKFLYNDNDKYGAGNVDEKDAPKEL
LDMLARAEREKK (SEQ ID No: 11)
SVKVKFLLNGEEKEVDTSKIRDVCRQGKNVKFLYNNNGKYGAGNVDEKDAPKEL
LEMLARAEREKK (SEQ ID No: 12).

Another advantageous property of the immunoglobulin-binding proteins according to the invention is that they are stable over a broad pH range. In particular, a protein according to the invention remains more than 80% active after a cycle of denaturation at pH=1 during 24 hrs followed by renaturation, as shown in the examples below. More than 80% of such a protein also remains active after 24 hours of incubation at pH=11, followed by renaturation. In the present text, a protein will be considered as "stable" under a certain pH condition if it still binds hIgG with at least 80% of its initial binding activity after (i) incubation during 24 hours at said pH, followed by (ii) a neutralization step, for example by adding 490 µl of an appropriate neutral solution (for example 20 mM Hepes, 150 mM NaCl, 0.005% P20, pH 7.4) to 10 µl of the protein/buffer solution. According to this definition, an immunoglobulin-binding protein according to the present invention is preferably stable at pH 1 to pH 11.

Another aspect of the present invention is a chimeric protein comprising an immunoglobulin-binding protein as above-described, coupled to a second protein moiety. The coupling between the two moieties of a "chimeric protein" according to the present invention can be either a genetic fusion (the chimeric protein is then a fusion protein), or a chemical binding. In the later case, the binding can be either covalent or non-covalent.

Examples of fusion proteins which can advantageously be obtained according to the present invention comprise fusions in which the immunoglobulin-binding protein moiety is fused, at its C- or N-terminal extremity, to a second moiety selected among:
- a reporter moiety such as a fluorescent protein (GFP, BFP, YFP, *etc.*) or an enzyme tag (luciferase, alkaline phosphatase, GST, β galactosidase, *etc*.), ...
- a binding moiety such as a peptide tag (His*6, FLAG, HA, myc, *etc*.), a DNA-binding domain, a hormone-binding domain, a tag for *in vivo* biotinylation (provided BirA is co-expressed) to enable oriented immobilization on a support (such as a protein chip), a poly-lysine tag (for example, Lys*6) for enabling oriented immobilization on a support (such as an affinity chromatography resin), ...
- a targeting moiety such as a signal sequence (for example, to address the chimeric protein to a specific compartment like periplasm or nucleus), ...

Advantageously, the fusion will encompass a peptide linker between the two moieties, which will be long enough to avoid inhibiting interactions between both moieties. The linker can optionally comprise a recognition site for a protease, if easy separation of both moieties is wanted. For example, the linker can comprise the sequence Glu-Asn-Leu-Tyr-Phe-Gln-Gly, which is the optimum recognition site for TEV protease (a highly site-specific cysteine protease that is found in the Tobacco Etch Virus). This enzyme has a high specificity and a high activity rate and triggers cleavage between the Gln and Gly residues of the above recognition site. It can hence be used, for example, for removing affinity tags from a purified chimeric protein according to the present invention.

Alternatively, as mentioned above, the two moieties are bound by chemical cross-linking. In particular examples of such chimeric proteins according to the invention, the immunoglobulin-binding moiety is bound to a second moiety selected among horse radish peroxidase (HRP), biotin, a fluorescent dye (AlexaFluor, FITC, *etc.*), a cysteine (bound far enough from the active site of the IgG binder, to enable binding of another compound through thiol chemistry), a radio-nuclide, *etc.*

The present invention also pertains to the use of an immunoglobulin-binding protein or of a chimeric protein as above-described, for performing a detection assay, for example an immuno-assay. Non-limitative examples of immuno-assays which can advantageously be performed with proteins according to the present invention are Western blots, ELISA, ELISpot assays, Immuno-precipitation (such as "pull-down"), Immuno-cytochemistry, Immuno-histochemistry, Flow cytometry, Multiplex assay, Radio-immuno assays, *etc.*

Immunoglobulin-binding proteins according to the invention can also be used for performing a capture assay. A particularly advantageous use of an immunoglobulin-binding protein or of a chimeric protein according to the present invention is affinity chromatography.

The invention is further illustrated by the following figures and examples.

### Legends to the figures

Figure 1: Schematic representation of wild type Sac7d (PDB code 1AZP).
   Residues involved in DNA binding and that were substituted are indicated.

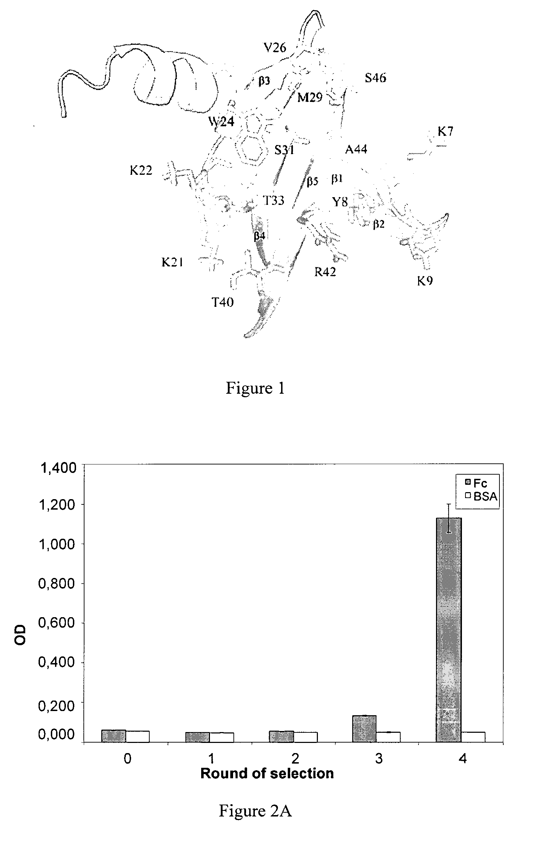

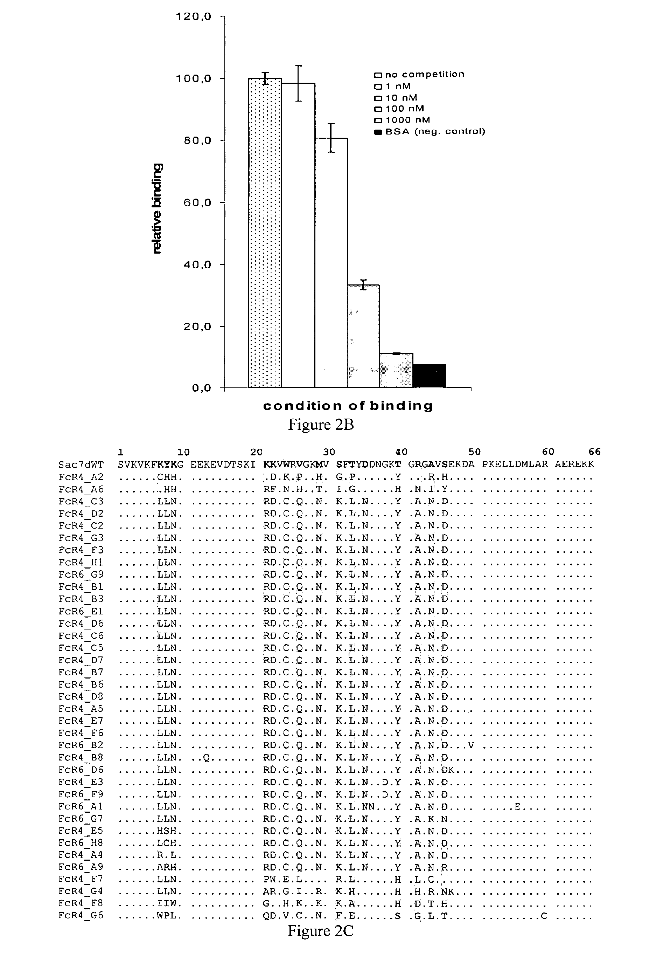

Figure 2: A) ELISA on selected pool after four rounds of selection. The selection pool was translated *in vitro* and tested for binding to Fc immobilized in an ELISA plate. Binding detection of the obtained binders was done using an anti-His-tag antibody conjugated to HRP. B) Competitions were carried out in parallel with pre-incubation of translated pools with free Fc at 1 nM, 10 nM, 100 nM and 1 µM. Immobilized BSA was used as negative binding control (no competition). C) Sequences of 37 selected Fc binders. The sequence of Sac7d is shown at the top of the figure. Residues common to the Sac7d and antibody-binding proteins are indicated by a dot. Positions that were programmed in the substitution scheme are highlighted in grey.
Figure 3: production and purification of 11 selected Fc binders from round 4 on a 50 ml scale. Proteins were separated by SDS-PAGE (15% acrylamide) and stained with Coomassie brilliant blue.
Figure 4: Size exclusion chromatography of Fc binder C3. The molecular mass standards, alcohol dehydrogenase (150 kDa), BSA (66 kDa) carbonic anhydrase (29 kDa), cytochrome C (12.4 kDa), aprotinin (6.5 kDa) are indicated.
Figure 5: Study of C3 specificity by ELISA. Tested IgG, and BSA as a negative control, were immobilized by adsorption in ELISA wells. C3 binding was detecting via its His-tag using an anti-His-tag antibody conjugated to HRP.
Figure 6: Affinity determination of clone C3 using SPR. hIgG1 was immobilized on a flow cell at low densities. Binding of the binder C3 to the immobilized target protein was compared to binding to a flow cell in which no IgG was immobilized. Increasing concentrations of the C3 were injected: 16 nM, 31 nM, 63 nM, 125 nM, 250 nM, 500 nM, 1000 nM. A dissociation constant of 94 nM was determined by performing a global fitting with Biaeval software (kₒₙ = 5.4 10⁵ M⁻¹ s⁻¹, k_{off} = 5.1 10⁻² s⁻¹). Fitted curves are plotted in black.
Figure 7: Sub-class specificity study of the C3 variant. Biotinylated C3 was immobilized on a streptavidin chip. Each sub-class was injected independently at a concentration of 500 nM.
Figure 8: Study of the specificity of C3 by western blot. A) A 8% SDS-PAGE gel was loaded with pure hIgG or spiked hIgG with exogenous proteins. B) After migration and transfer to nitrocellulose membrane, C3 labeled with AlexaFluor 680 was used for detection of hIgG. 1) *E. coli* crude extract, 2) hIgG + E. *coli* crude extract, 3) hIgG, M) ladder, 4) DMEM/CS, 5) hIgG + DMEM/CS.
Figure 9: Study of the effect of pH on structuration of C3, WT Sac7d and Protein A. Proteins at 0.33 mg/ml were incubated at room temperature overnight in a solution buffered at each pH value from pH = 0 to pH = 13. The unfolded fraction of protein was calculated according to the residual ellipticity that was measured by circular dichroism.
Figure 10: Comparative study of the kinetic of deactivation for C3 (A and B) and Protein A (C and D) at different pH. Residual activities of binding proteins were monitored by SPR after incubation in a neutralizing buffer as indicated in material and methods.

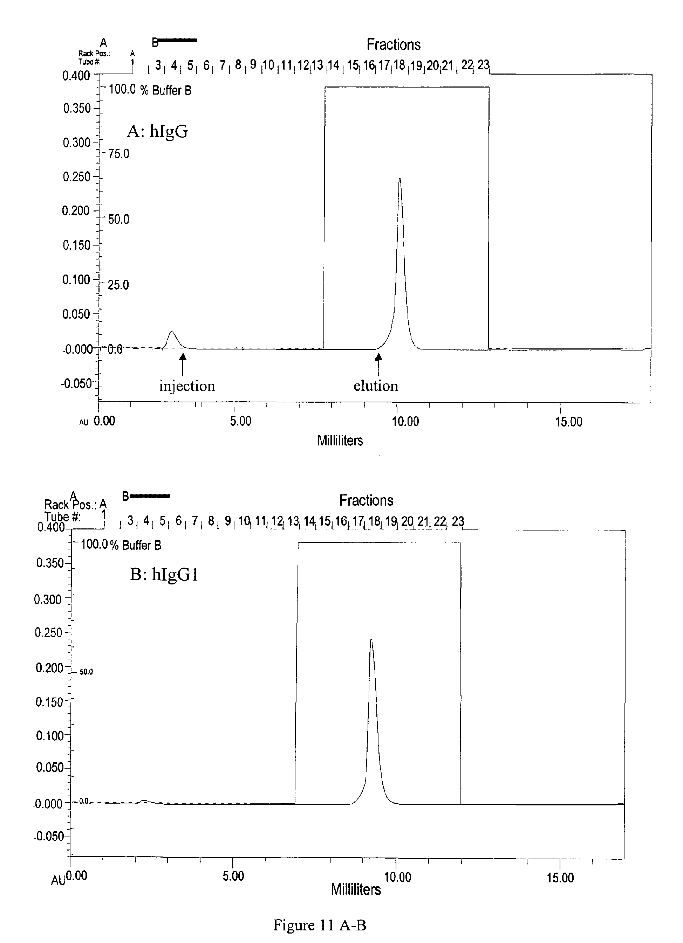

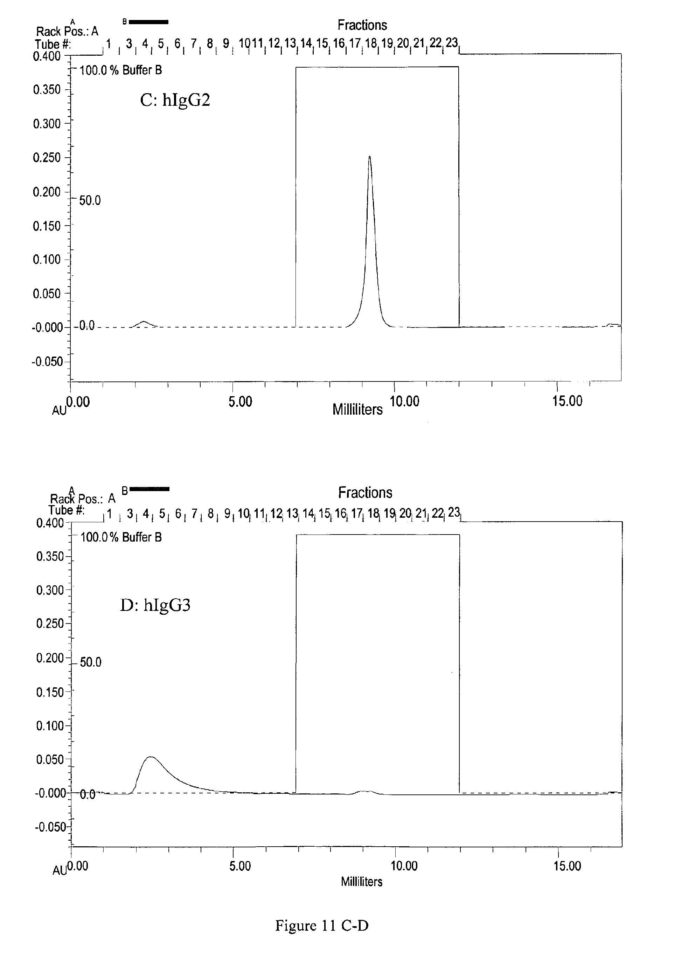

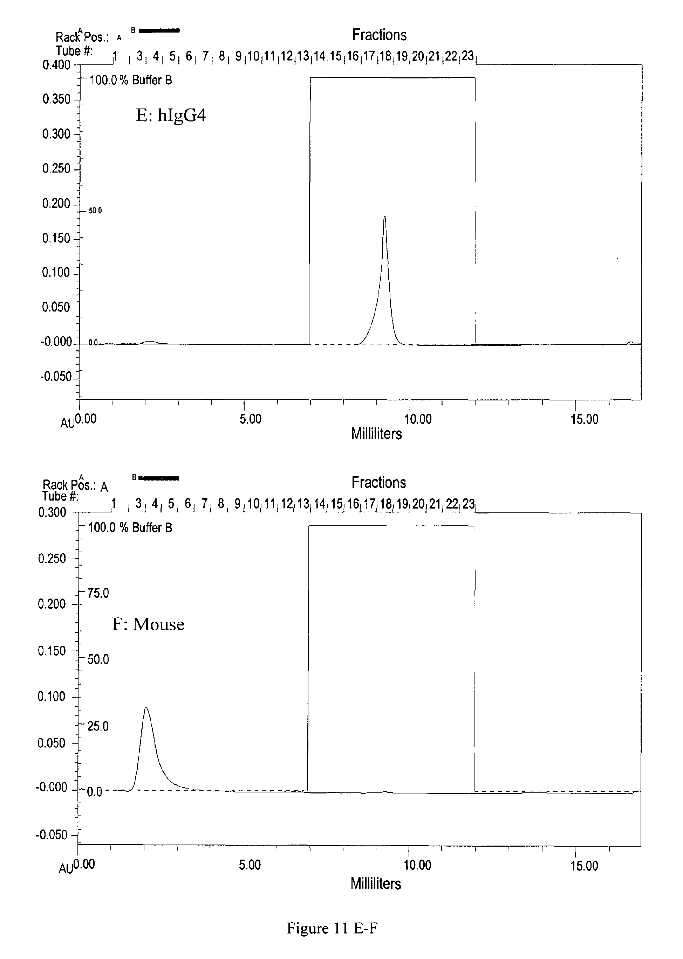

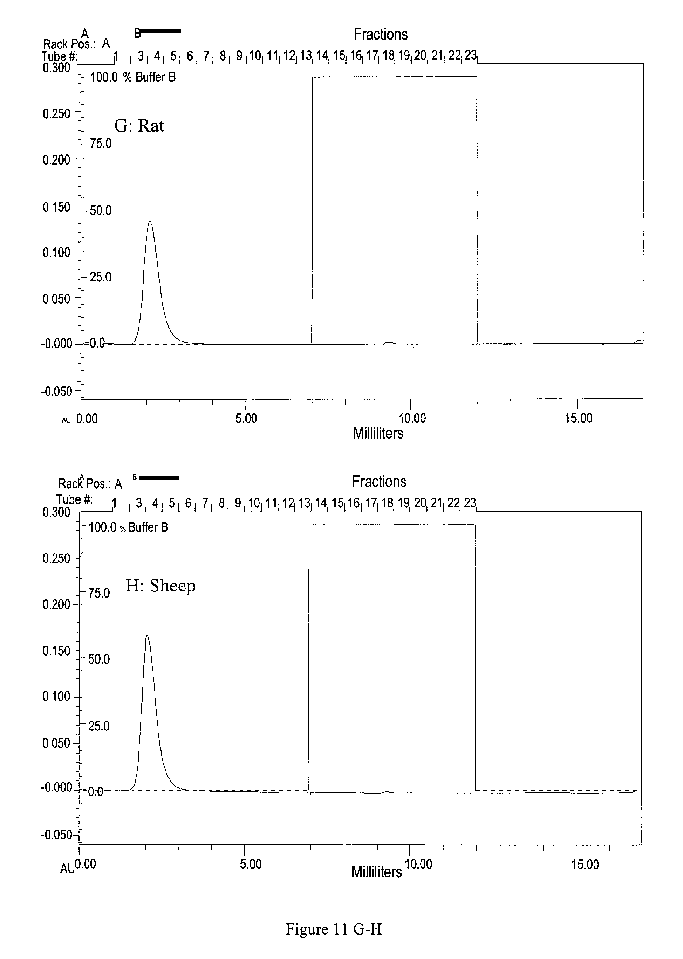

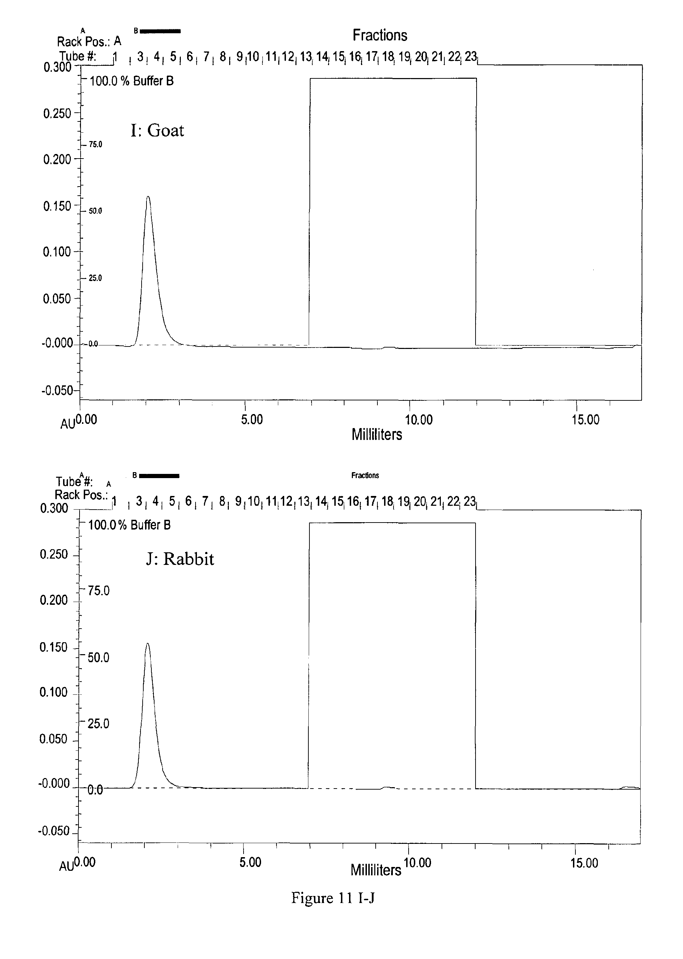

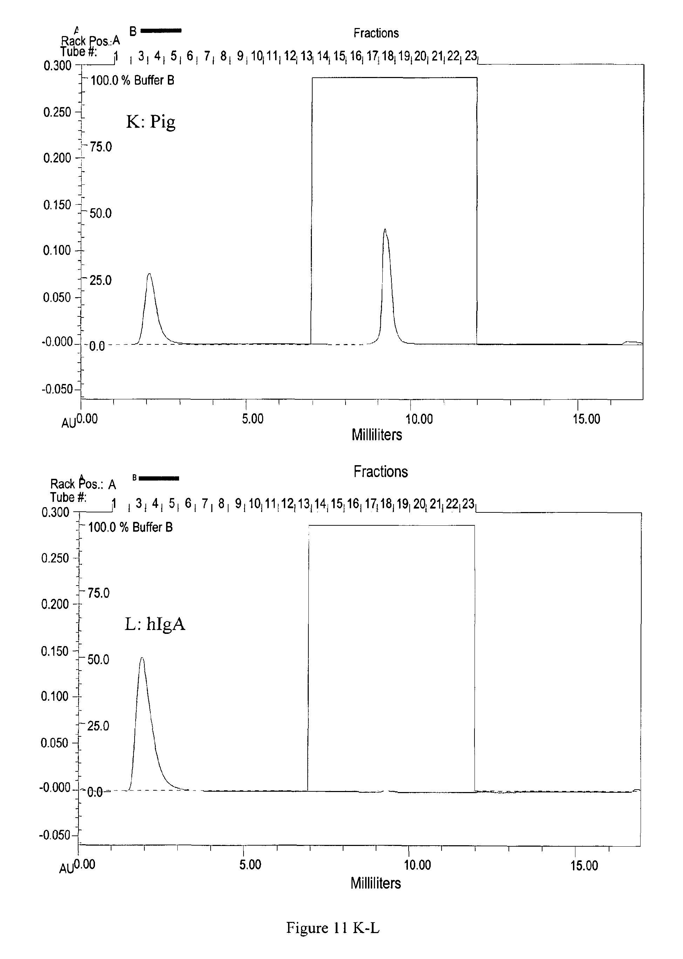

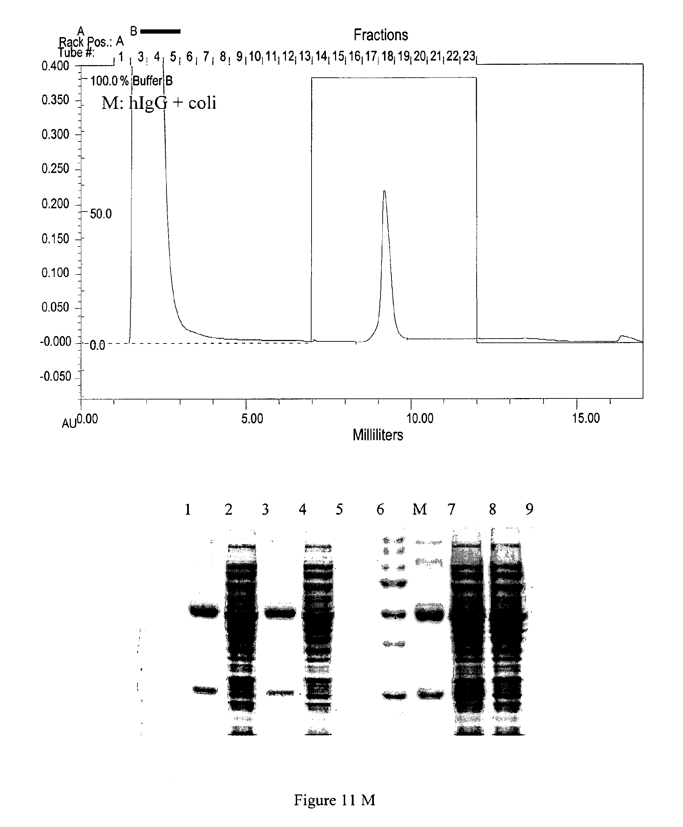

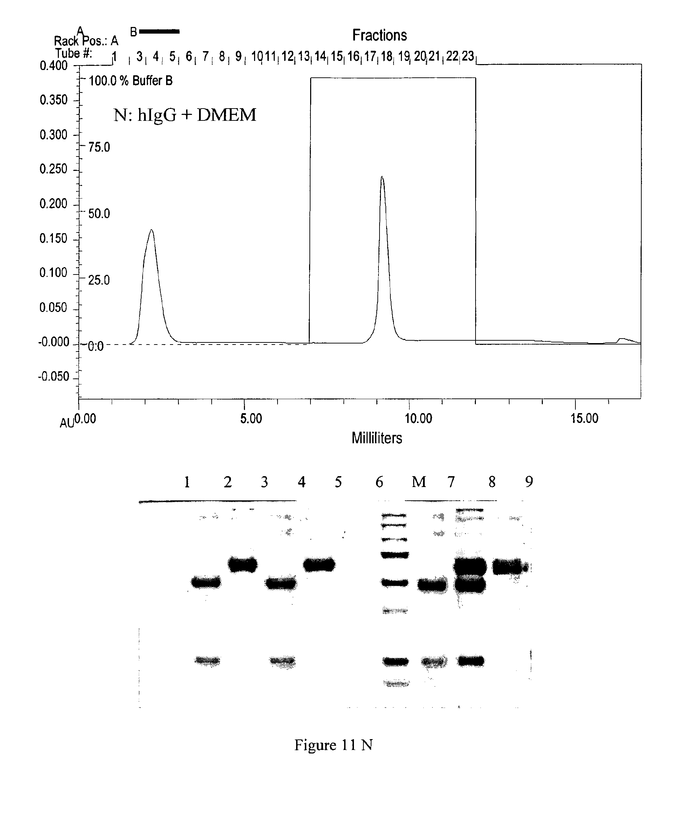

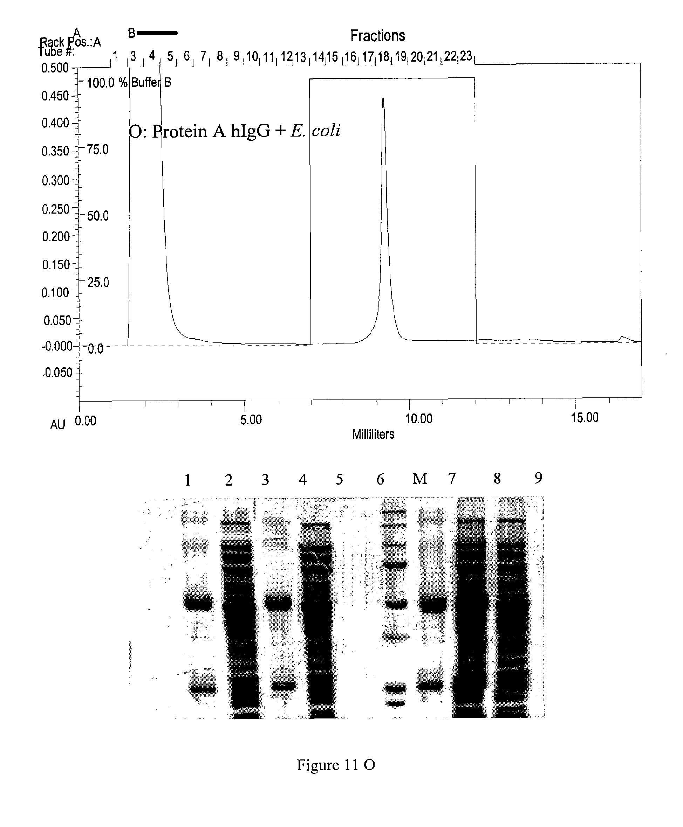

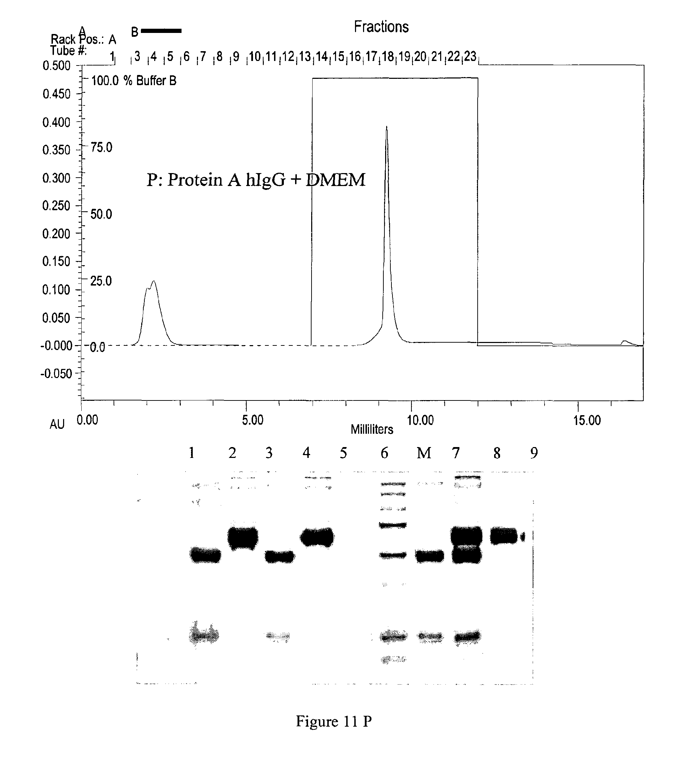

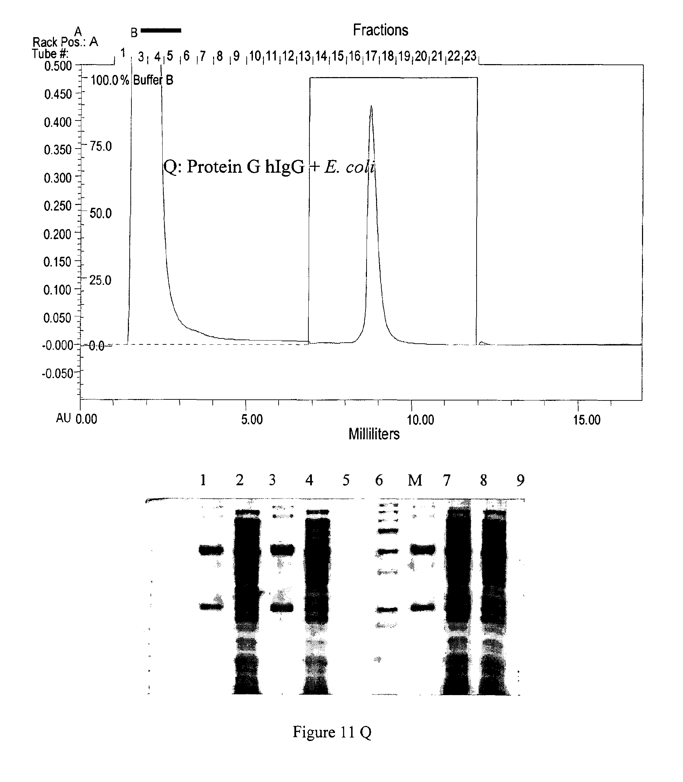

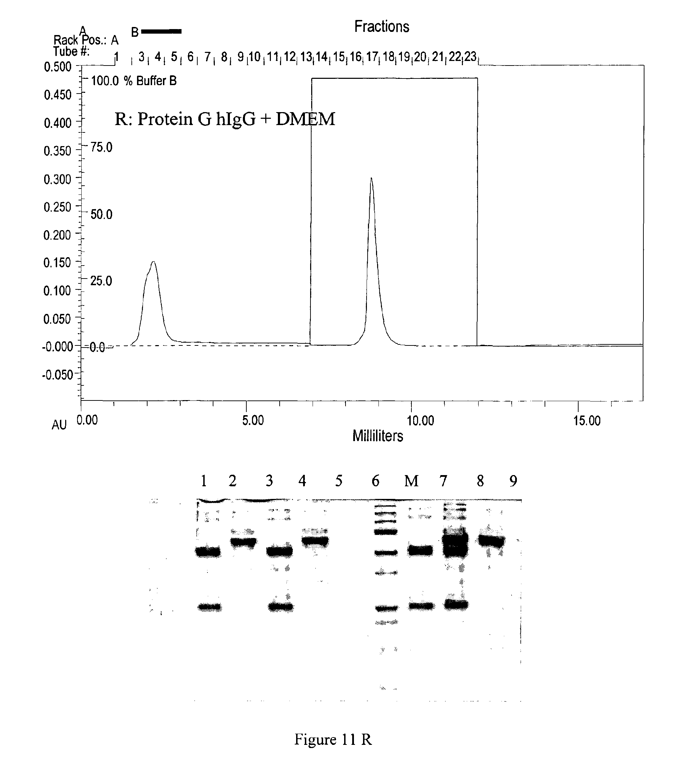

Figure 11: Chromatograms of the affinity purification of the four hIgG subclasses. Each pure hIgG was injected on a C3 column. After washings with running buffer, elution was done with a glycine buffer pH 2.5. **A-R:** The OD at 280 nm was measured to monitor the chromatography. **M** (lower panel): SDS-PAGE analysis (under reducing conditions) of affinity purifications using a C3 column : pure hIgG injected (1: flow through, 2: elution), E. *coli* crude extract + hIgG (3: flow through, 4: elution), E. *coli* crude extract (5: flow through, 6: elution). As controls samples that were not submitted to chromatography were loaded on lane 7 (pure hIgG), 8 (E. *coli* crude extract + hIgG), 9 (*E*. *coli* crude extract). **N** (lower panel): SDS-PAGE analysis (under reducing conditions) of affinity purifications using a C3 column : pure hIgG injected (1: flow through, 2: elution), DMEM + 10% calf serum + hIgG (3: flow through, 4: elution), DMEM + 10% calf serum (5: flow through, 6: elution). As controls samples that were not submitted to chromatography were loaded on lane 7 (pure hIgG), 8 (DMEM + calf serum + hIgG), 9 (DMEM + 10% calf serum). **O** (lower panel): SDS-PAGE analysis (under reducing conditions) of affinity purifications using a Protein A column : pure hIgG injected (1: flow through, 2: elution), E. *coli* crude extract + hIgG (3: flow through, 4: elution), E. *coli* crude extract (5: flow through, 6: elution). As controls samples that were not submitted to chromatography were loaded on lane 7 (pure hIgG), 8 (E. *coli* crude extract + hIgG), 9 (E. *coli* crude extract). **P** (lower panel): SDS-PAGE analysis (under reducing conditions) of affinity purifications using a Protein A column : pure hIgG injected (1: flow through, 2: elution), DMEM + 10% calf serum + hIgG (3: flow through, 4: elution), DMEM + 10% calf serum (5: flow through, 6: elution). As controls samples that were not submitted to chromatography were loaded on lane 7 (pure hIgG), 8 (DMEM + calf serum + hIgG), 9 (DMEM + 10% calf serum). **Q** (lower panel): SDS-PAGE analysis (under reducing conditions) of affinity purifications using a Protein G column : pure hIgG injected (1: flow through, 2: elution), E. *coli* crude extract + hIgG (3: flow through, 4: elution), E. *coli* crude extract (5: flow through, 6: elution). As controls samples that were not submitted to chromatography were loaded on lane 7 (pure hIgG), 8 (*E*. *coli* crude extract + hIgG), 9 (E. *coli* crude extract). **R** (lower panel): SDS-PAGE analysis (under reducing conditions) of affinity purifications using a Protein G column: pure hIgG injected (1: flow through, 2: elution), DMEM + 10% calf serum + hIgG (3: flow through, 4: elution), DMEM + 10% calf serum (5: flow through, 6: elution). As controls samples that were not submitted to chromatography were loaded on lane 7 (pure hIgG), 8 (DMEM + calf serum + hIgG), 9 (DMEM + 10% calf serum).

### Examples

The experimental results described below have been obtained by using the following materials and methods.

### Materials and Methods

### General molecular biology

Enzymes and buffers were from New England Biolabs (USA) or Fermentas (Lithuania). Oligonucleotides were from Eurofins-MWG (Germany). All PCRs (polymerase chain reactions) were performed using Vent polymerase if not indicated in the text. The cloning and expression vector for the wild-type Sac7d and selected mutants was pFP1001. This vector is a derivate of pQE3 0 from Qiagen (Germany) in which we replaced by PCR the stop codon TGA by two stop codons TAATGA using the following oligonucleotides QE_STOP_F (GCAGCCAAGCTTAATTAATGACTGAGCTTGGACTCCTGTTG) (SEQ ID No: 13) and QE_STOP_R (CGCCAAGCTAGCTTGGATTC) (SEQ ID No: 14). The PCR product was cloned into pQE30 vector using HindIII and NheI restriction sites. A clone with the expected sequence was used for subsequent expressions.

### Synthesis of the DNA encoding the wild type Sac7d

This synthesis has already been described in WO 2008/068637 A2 (page 13)

### Generation of combinatorial libraries

The generation of combinatorial libraries has already been described in WO 2008/068637 A2 (pages 13 and 14). Library 14 was used in the present study.

### Ribosome display selection rounds

For selection experiments, biotinylated target protein was used. The biotinylation was performed by incubation of a 10 µM solution of human IgG-Fc fragment (Bethyl Laboratories) with a 20-fold molar excess of Sulfosuccinimidyl-6-(biotinamido) hexanoate (Sulfo-NHS-LC-LC-Biotin, Pierce) in PBS on ice for 1 h. The biotinylated proteins were buffer-exchanged using protein desalting spin columns from Pierce equilibrated in TBS. The degree of biotinylation was determined using the HABA assay (Sigma) as 1.7 molecules biotin per protein molecule. Biotinylated target proteins were bound to immobilized neutravidin or streptavidin alternatively from round to round in a Maxisorp plate (Nunc) and selections by ribosome display were performed at 4°C essentially as described (Binz et al., 2004) with some modifications. Briefly, after each round of selection the eluted mRNA was reverse-transcribed to cDNA and amplified using SDA_MRGS and ScepRev (TCGGCCCCCGAGGCCATATAAAGCTTTTTCTC) (SEQ ID No: 15) primers. The PCR product was desalted on a Nuclespin ExtractII column (Macherey-Nagel) and used for a PCR assembly with TolAlinker DNA fragment (see above). This generated a full length ribosome display construct with newly added 5'- and 3'- flanking regions to minimize loss of clones due to degradation of the extremities of mRNA during its manipulation. Progresses of selections were checked by monitoring the amounts of RT-PCR products from round to round.

### Analysis of selected pools and isolated clones

After four or six rounds, *in vitro* translated selected pools were tested by ELISA as described using directly coated target protein in a Maxisorp plate and using from 1 nM to 1 µM of free Fc protein as competitor. Detection of binding to Fc was performed using the RGS His antibody HRP conjugate (Qiagen) and a solution of 1 mg/ml o-Phenylenediamine substrate (Sigma).

The RT-PCR products from selected pools were cloned into pFP1001 vector using BamHI and HindIII restriction sites and the resulting ligations were used to transform *E. coli* DH5α Iq strain. Clones were picked from Petri dish to inoculate a deep-well plate containing 1.5ml of LB medium per well (100 µg/ml ampicillin, 25 µg/ml kanamycine, 1 % glucose). After overnight culture at 37°C with shaking at 250 rpm, 0.2 ml of each well from this master plate was used to inoculate an other deep-well plate containing 1.4 ml 2YT medium (100 µg/ml ampicillin, 25 µg/ml kanamycine, 0.1 % glucose) per well. The plate was then incubated at 37°C for 2-3h with shaking (750 rpm). The expression was induced with the addition of 0.5 mM IPTG and O/N incubation at 30°C with shaking (750 rpm). Cells were pelleted with a centrifugation step (2000g) and supernatants discarded. Proteins were extracted with 50 µl BugBuster (Novagen) containing 5µg/ml DNase I (Sigma) per well with shaking at 750 rpm for 30min, then 250 µl of TBS pH 7.4 (20 mM Tris-HCl, 150 mM NaCl) were added. Cell debris were pelleted with a centrifugation step (2000g). For ELISA (enzyme-linked immunosorbent assay) screening, 100 µl of each supernatant were used to test the binding on target proteins, and BSA as negative control, coated into a Maxisorp plate. The detection was performed using the RGS His antibody HRP conjugate (Qiagen) that detects only the RGS-(His)6-tag from binders and a solution of 1 mg/ml OPD substrate (Sigma) and absorbance at 450 nm was measured. All incubation steps were carried-out in TBS pH 7.4 with 0.1% Tween 20. Positive clones were sequenced by standard sequencing techniques.

### Protein production for screening by SPR (surface plasmon resonance)

To perform the screening of binders based on their time of dissociation from hIgG, clones were expressed in *E. coli* DH5α Iq strain. Two hundreds microliters of an overnight preculture (LB medium, 100 µg/ml ampicilin, 25 µg/ml kanamycin, 1% glucose, 37°C,) in deepwell were used to inoculate 1.4 ml culture (2YT, 100 µg/ml ampicilin, 25 µg/ml kanamycin, 0.1% glucose, 37°C, 750 rpm) in deepwell. Expression was induced at OD600 = 1.0 by addition of 0.5 mM IPTG and cultures were incubated for 19h at 30°C (750 rpm). Cells were harvested by centrifugation (2000 g) and proteins were extracted in deepwells by resuspension of cells in 1.0 ml TBS pH 7.4 containing 25 mM imidazole, BugBuster and containing 5µg/ml DNase I. After 1h shaking at room temperature, the deepwell was centrifuged to pellet cell debris. Supernatants were purified on microspin columns containing 100 µl Ni-Fast Flow Chelating Sepharose (General Electric) equilibrated with TBS pH 7.4 containing 25 mM imidazole. Resin was washed 4 times with the loading buffer and purified proteins were eluted with 150 µl TBS pH 7.4 and 250 mM imidazole.

### Protein production and purification of binders and wild-type Sac7d

For SPR, Western blots, affinity chromatography and circular dichroism experiments, and depending on the needs the binders were expressed in DH5α Iq *E. coli* strain in a 50 ml to 1.0 liter scale and purified as described below for the 1 liter scale. Fifty milliliters of an overnight preculture (LB medium, 100 µg/ml ampicillin, 25 µg/ml kanamycin, 1% glucose, 37°C) were used to inoculate 1 liter culture (2YT, 100 µg/ml ampicilin, 25 µg/ml kanamycin, 0.1 % glucose, 37°C). Expression was induced at OD600 = 0.8-1.0 by addition of 0.5 mM IPTG and cultures were incubated O/N (variant of Sac7d) or 2h (wt Sac7d) at 30°C (220 rpm). Cells were harvested by centrifugation and resuspended in 30 ml TBS pH 7.4 (20 mM Tris, 500 mM NaCl) at RT containing 25 mM imidazole. Cells were lyzed with an Avestin Emulsiflex homogenizer and cell debris were discarded with a centrifugation step. Proteins were purified on a 5 ml HiTrap chelating column (GE Healthcare) equilibrated with TBS pH 7.4 containing 25 mM imidazole. Elution was performed with TBS pH 7.4 and 250 mM imidazole. Proteins were further purified with a Biologic Duoflow system (Biorad) connected to a size-exclusion chromatography Superdex 75 16/60 gel filtration column (GE Healthcare). The column was equilibrated with TBS pH 7.4 (20 mM Tris, 500 mM NaCl). Calibration of the gel filtration column was done by injecting Aprotinin (6.5 kDa), Cytochrome C (12.4 kDa), Carbonic anhydrase (29 kDa), BSA 66 kDa), Alcohol dehydrogenase (150 kDa) as molecular weight gel filtration calibration standards.

### Immunoglobulins

Ig used in this study were purchased from Fluka AG (Buchs, Switzerland): hIgG (*i.e.,* IgG pool from human serum containing hIgG1, hIgG2, hIgG3, hIgG4 : reference number 56834); and from Sigma-Aldrich: hIgG1 (reference number : I5029), hIgG2, hIgG3, hIgG4, IgG from mouse, rat, sheep, goat, rabbit, pig and hIgA.

### Surface Plasmon resonance

SPR was measured using a BIAcore 3000 instrument at 25°C. hIgG or hIgG1 (1500-2000 RU) was immobilized on flow cells of a CM5-chip. The running buffer was HBSEP pH 7.4 (20 mm Hepes, 150 mm NaCl, and 0.005% P20). Regeneration was performed with glycine 10 mM, pH 2.5.

The screening and the ranking of binders according to their off-rate were performed using micro IMAC purified proteins (see above) diluted 1/5 in running buffer prior injections for kinetics measurements at a flow rate of 60 µl/min.

Kinetic measurements for affinity determinations of hIgG binders were performed with size-exclusion purified proteins injected at concentration ranging from 15 nM to 1000 nM. Data evaluation was done using Scrubber2 (Biologic software) and BIAeval software (BIAcore) using the 1:1 Langmuir binding model and a global fitting procedure (Karlsson and Falt, 1997).

Affinities of each hIgG isotype for immobilized biotinylated C3 (250 RU) on flow cells of a SA-chip were measured in a single-cycle mode kinetic also called "Kinetics titration series" for 3 min at 60 µl/min allowing a 4 min dissociation phase. The resulting sensorgrams were fitted using a mathematical program based on single-cycle kinetic model (Kinetic titration series model) implemented in BIAeval 4.1 software (BIAcore) (Karlsson et al., 2006).

### Study of specificity by ELISA

One hundred microliters of purified C3 at 10 µM was used for ELISA to test its binding on immunoglobulins, and BSA as negative control, coated into a Maxisorp plate. The detection was performed using the anti-RGS His antibody HRP conjugate (Qiagen) that detects the RGS-(His)6-tag from binders and a solution of 1 mg/ml o-Phenylenediamine substrate (Sigma) and absorbance at 450 nm was measured. All incubation steps were carried-out in TBS pH 7.4 with 0.1% Tween 20.

### Labeling of C3 with AlexaFluor 680

Fifty microliters of C3 (2 mg/ml in 0.1 M NaHCO₃ pH7.5) were mixed with 3 µl of Alexa 680 succinimidyl ester (10 mg/ml in dimethyl sulfoxide) and incubated 1h at RT. The labeling was stopped by desalting the reaction mix using a protein desalting spin columns (Pierce) equilibrated in TBS.

### Study of specificity by Western blot

Ten micrograms of hIgG were loaded on a non reducing 8% SDS-PAGE gel. Skiped hIgG were prepared with DMEM + 10 % calf serum (*i*.*e*., 40 % of DMEM final concentration in the sample loaded) or with an *E. coli* crude extract prepared from DH5α Iq strain (5X concentrated from initial culture in the sample loaded). The proteins in each sample were separated by SDS-PAGE and transferred onto nitrocellulose. The detection of hIgG was performed using 2 µg/ml C3 labeled with AlexaFlor 680 in TBS containing 0.1% Tween-20 and 1% BSA. Bands were detected using an infra red Odyssey scanner (LI-COR).

### Study of the effect of pH on C3 stability by circular dichroism

Proteins in TBS 7.4 were diluted to 0.33 mg/ml in buffered solution or a strong acid corresponding to each pH unit from 0 to 13. Except for HCl or NaOH solutions, the following solutions were adjusted to the required pH with HCl or NaOH: pH 0 = 1 M HCl, pH 1 = 0,1 M HCl, pH 2 = 50 mM NaH₂PO₄, pH 3 = 50 mM NaH₂PO₄, pH 4 = 50 mM acetic acid, pH 5 = 50 mM acetic acid, pH 6 = 50 mM MES, pH 7 = 50 mM phosphoric acid, pH 8 = 50 mM NaH₂PO₄ , pH 9 = 50 mM Tris base, pH 10 = 50 mM methylamine, pH 11 = 50 mM methylamine, pH 12 = 50 mM NaH₂PO₄, pH 13 = 0,1 M NaOH.

Protein sample were incubated O/N at room temperature and residual ellipticity was measured at 222 nm on a JASCO J-810 instrument (Jasco, Japan), using a quartz cell with a path length of 0.2 cm (Hellma, Germany). The temperature was maintained at 20°C with a programmable Peltier single cell holder.

### Kinetics of deactivation of C3 and Protein A monitored by SPR

hIgG1 (1600 RU) was immobilized by amine coupling chemistry on flow cells of a CM5-chip. C3 protein (250 µM) and Protein A (40 µM) were independently incubated with 90 µl of buffer solution at pH 1, 2, 3, 9, 11, 12 or 13. The solution used were for pH 1 = 0,1 M HCl, pH 2 = 50 mM NaH₂PO₄, pH 3 = 50 mM NaH₂PO₄, pH 9 = 50 mM Tris base, pH 11 = 50 mM, pH 12 = 50 mM NaH₂PO₄, methylamine, pH 13 = 0,1 M NaOH. Kinetic of deactivation by pH was stopped by neutralization of 10 µl of the protein/buffer mix into 490 µl of the SPR running buffer HBSEP pH 7.4 (20 mm Hepes, 150 mm NaCl, and 0.005% P20). The residual functional protein able to bind hIgG was measured by SPR by injecting C3 at 500 nM and Protein A at 80 nM. Regeneration was performed with glycine 10 mM, pH 2.5.

### Affinity chromatography using C3

Thirteen milligrams of purified C3 was dialyzed O/N against PBS and was injected on a 1 ml HiTrap NHS-activated HP column (General Electric) previously flushed with 6 ml of 1 mM HCl. Immobilization occurred at room temperature for 1h. After washing the column and deactivating any remaining active groups with 0.5 M ethanolamine (0.5 M NaCl, pH 8.3) and 0.1 M acetate (0.5 M NaCl, pH 4) for 30 min at room temperature, the column was equilibrated with TBS pH 7.4 (20 mM Tris, 500 mM NaCl) and was ready to use for purification.

About 125 µg of each IgG or IgA were used to study the NHS-C3 immobilized column. Spiked Ig were prepared with DMEM + 10 % calf serum (40 % of DMEM final concentration in the sample) or with an *E. coli* crude extract prepared from DH5α Iq strain (5X concentrated from initial culture in the sample). Non specific proteins were washed away with 5 ml of running buffer and Ig was eluted with an acidic pH jump using a 100 mM glycine buffer (pH 2.5, 150 mM NaCl). Purity of the eluted protein was checked by loading fractions on a reducing SDS gel.

### Results and discussion

### Example 1: Selection of immunoglobulin binders

The inventors have used the previously described combinatorial library of Sac7d (Mouratou et al., 2007) as the source of potential binders. This library of about 3.0 10¹² Sac7d variants corresponds to the random substitution of 14 residues that compose the potential binding area (Fig. 1).

To isolate variants able to bind to human IgG independently of their hypervariable regions, the selection was performed using a purified Fc fragment from a polyclonal human antibodies pool as the target. An ELISA plate coated alternatively from round to round with streptavidin or neutravidin was used to immobilize biotinylated Fc and six rounds of selection by ribosome display were done. The enrichment for Fc binders was monitored by ELISA to test progress of the binding activity during selection (Fig. 2a). Enrichment was already observed after only three rounds of selection, but it increased by a factor of about 11 between the third and the fourth round as expected for a positive selection. Binding could not be detected on immobilized BSA. A competition ELISA after the fourth round (Fig. 2b) suggested that the pool contained specific binders for human Fc with affinities around one hundred nanomolar.

### Example 2: Characterization of Fc binders

### Sequence analysis, expression and purification of selected binders

The enriched pool was cloned into an expression plasmid and the binders produced with His-tag in the cytoplasm of *E*. *coli* strain DH5α-Iq. Crude extracts from 80 individual clones assayed by ELISA with immobilized Fc or BSA showed significant and specific Fc binding for about 52 clones (∼65% positive). Clones were used for microexpression and immobilized metal ion affinity chromatography purification to identify those with highest affinities. The proteins were then screened by surface plasmon resonance (SPR) with a chip coated with Fc fragment. Thirty seven clones were chosen for their slowest kinetic of dissociation and their DNA sequence analysis was performed. This revealed that there were 17 different clones (Fig 2c). The sequence corresponding to the C3 clone was found 20 times suggesting a convergence of the selection. Ten variants differed from C3 by 1 to 4 mutations. All clones from C3 family had an aspartate to asparagine substitution at position 35 (although this position had not been initially randomized), suggesting a positive effect for its selection. Finally, 6 unique clones were found different from the C3 family.

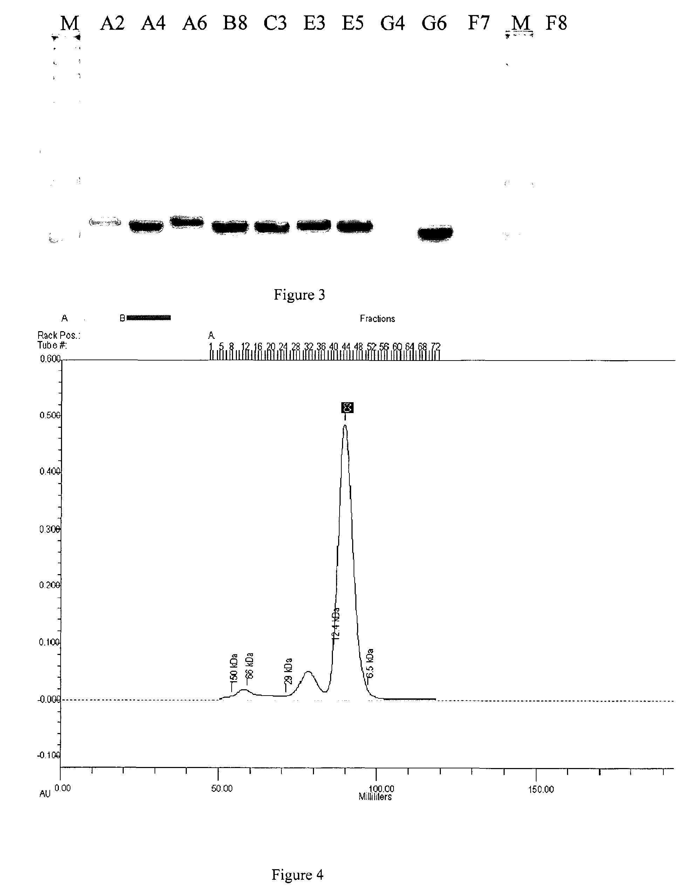

A subset of 11 different representative clones was produced in flask on a 50 ml scale. The binders accumulated in large amounts in the *E. coli* cytoplasm at 30°C after overnight growth and could be purified to homogeneity in two steps by IMAC and gel filtration with yields up to 200 mg per liter of shake flask culture. The proteins ran on a 15% acrylamide SDS-PAGE gel at the position expected for their calculated molecular masses (Fig. 3). Clones F7 and F8 were expressed with lower yields (31 and 6 mg per liter of shake flask culture, respectively), while the clone G4 was nearly not detectable after purification. Proteins eluted from gel filtration column as a symmetric peak (Fig. 4) with an apparent molecular weight of about 9.2 kDa as expected for monomeric species (theoretical molecular mass = 9.1 kDa).

### Example 3: Binding properties of selected binders

### Affinity determination

According to analysis of the dissociation phases, eight clones with the slowest off-rates were chosen for detailed affinity determinations of the monovalent proteins by SPR (Table 1). The chip was coated with hIgG (Fluka, reference number 56834), indicating that the selected binders were able to recognize Fc even in presence of Fab regions. This analysis also revealed that the dissociation constants of all binders were in the nanomolar range (Table 1). H8 had the highest apparent affinity (KD ∼ 34 nM) and E3 the lowest apparent affinity (KD ∼ 350 nM).

**Table 1: Affinities and binding kinetics of binders**

| binder | kₒₙ (10⁵ M⁻¹s⁻¹) | k_{off} (10⁻² s⁻¹) | K_{D} (nM) |
|---|---|---|---|
| H8 | 8.8 | 3.0 | 34 |
| A4 | 9.1 | 4.6 | 50 |
| D6 | 10.7 | 6.0 | 56 |
| B8 | 9.0 | 6.6 | 72 |
| C3 | 8.9 | 6.6 | 74 |
| E5 | 9.2 | 7.0 | 76 |
| A9 | 5.3 | 8.1 | 154 |
| E3 | 3.4 | 11.7 | 350 |

### Study of specificity

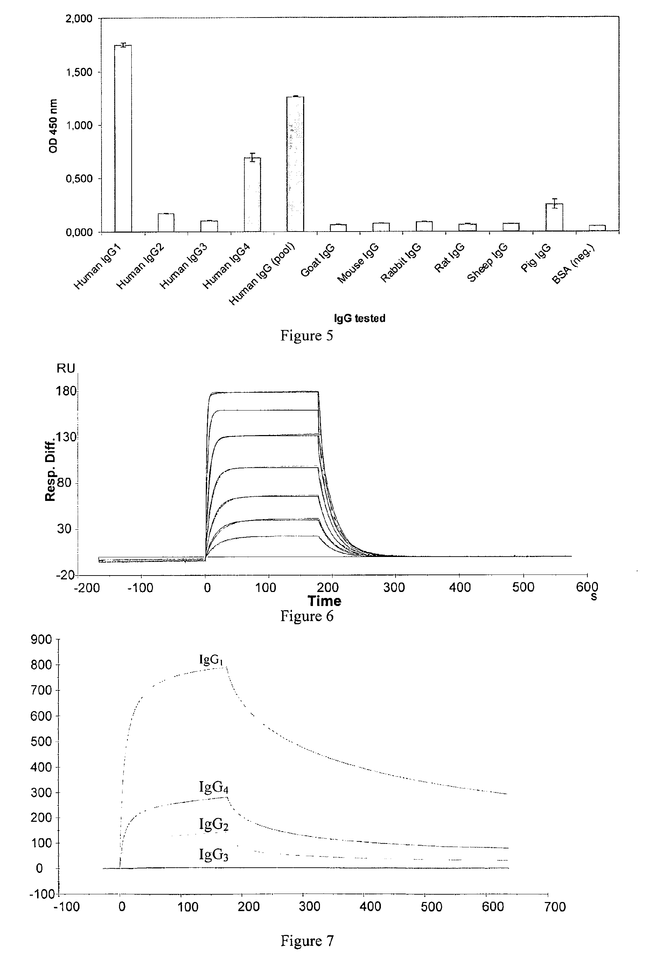

The clone C3 was chosen for further studies as it is the most represented clone in the pool of sequences and it has an affinity in the upper range of the affinities compatible with affinity chromatography applications (Graves and Wu, 1974). An ELISA with the four purified hIgG sub-classes indicated that C3 was able to interact with hIgG with decreasing strength in the following order: hIgG1 > hIgG4 >> hIgG2 > hIgG3 (Fig. 5). Nearly no interaction was detected between C3 and hIgG3 and only a weak signal was observed for hIgG2. In addition, no interaction could be detected by ELISA between C3 and streptavidin or neutravidin, proteins that were used during selections (data not shown). These results confirm there is a stronger interaction for hIgG1 than for the other human subclasses. The ELISA indicated also that C3 is able to recognize two or three hIgG subclasses but not IgGs from several other organisms tested, except IgG from pig, suggesting a narrow specificity (Fig. 5). The interaction between C3 and hIgG1 was confirmed by SPR experiments with hIgG1 immobilized on the chip (Fig. 6).

To confirm results obtained by ELISA and to have a better understanding of interactions between C3 and various IgGs, a SPR analysis was performed. Biotinylated C3 was immobilized on a streptavidin chip and IgGs were injected to monitor interactions. Figure 7 shows that interaction was stronger for hIgG1 than for hIgG4 and hIgG2, while no interaction could be detected for hIgG3. Among IgG from other species, only a weak interaction could be detected for pig. No interaction could be detected between C3 and hIgA or hIgM (not shown). These results confirm that C3 has a narrow specificity.

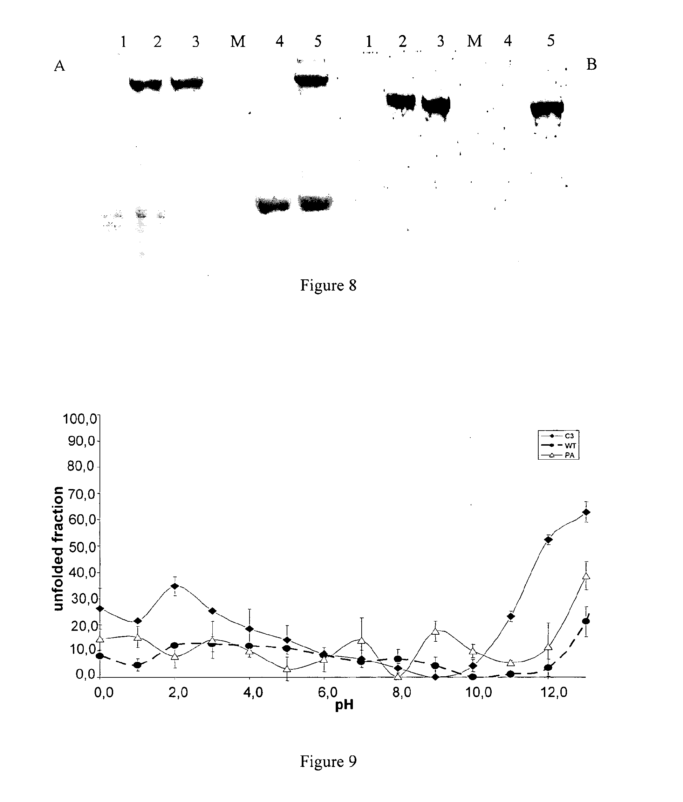

Next, the inventors investigated whether C3 is able to discriminate hIgG from other proteins by western blot. C3 was labeled with Alexa-Fluor 680 and used to detect hIgG spiked either with an E. *coli* total lysate or Dulbecco's Modified Eagles Medium supplemented with 10% calf serum (DMEM/CS). Analysis of the membrane showed that the only recognized proteins were hIgG in both cases, confirming that C3 had a high selectivity (Fig. 8).

### Example 4: Study of the pH stability of C3

To investigate whether C3 was stable enough to resist to extreme pH conditions generally used during affinity chromatography, C3 was submitted to overnight incubations at pH ranging from 1 to 13. Circular dichroism measurements at 222 nm indicated that C3 was mostly stable from pH 6 to pH 10 (Fig. 9). Denaturation occurred progressively for decreasing pH under 6 to 2. However, the CD signal after 24h at pH 0 was still about 75-80% of the signal at pH 9, suggesting that C3 retained the acidophilic property of Sac7d. For pH higher than 10, CD signal decreased reflecting an extensive denaturation at pH 13. A comparison with Sac7d showed that C3 stability was mainly altered for alkaline pH. Sac7d had a pH stability profile similar to the one of Protein A, suggesting that Sac7d, although being an acidophilic protein, is able to resist quite well to long alkaline treatments.

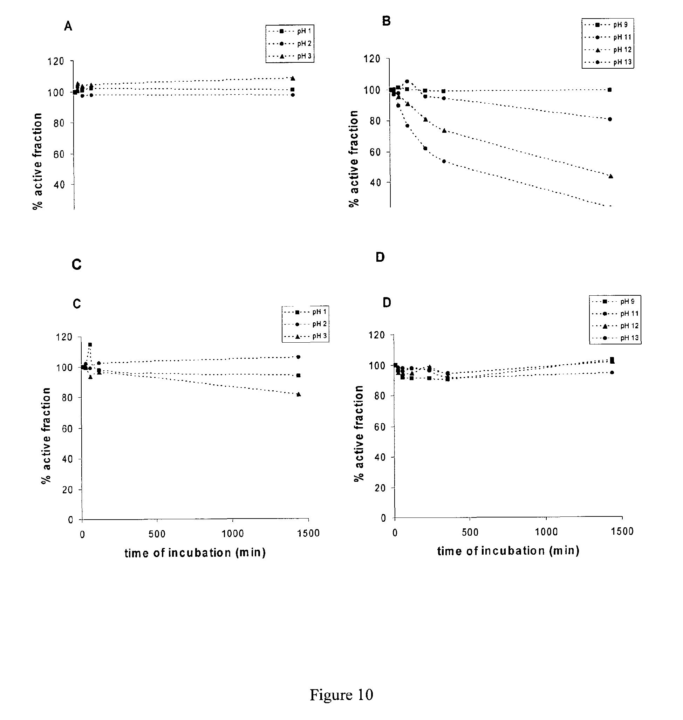

For this study at equilibrium, the circular dichroism signal at 222 nm monitors the degree of structuration of α-helixes in the proteins, but does not give information about their function and the reversibility of inactivation. To obtain more precise description of the denaturation, the fraction of C3 that remains active after a cycle of denaturation/renaturation was quantified by SPR. This was done by incubating C3 for a given time in denaturing buffers and then in a renaturation buffer. Nearly all C3 remained active at low pH (Fig. 10A), supporting the previous observation by circular dichroism (Fig. 9). Under alkaline conditions, a kinetic of inactivation was observed. About 50% and 20% of C3 remained active after overnight incubation at pH 12 and pH 13, respectively. The inactivation observed after overnight incubation at pH 11 was much slower since more than 80% of C3 was found active while C3 was 100% active at pH 9 (Fig. 10B). These data suggested that C3 is irreversibly altered under alkaline conditions, and probably not only at the α-helix level which is away from the potential binding area. A comparison with Protein A under same conditions shows that C3 is more stable than Protein A under acidic conditions (Fig. 10C). However, C3 is less stable than Protein A under alkaline conditions (Fig. 10D).

### Example 5: Affinity chromatography using C3

Next, the inventors investigated the use of C3 as a reagent for affinity chromatography. C3 was immobilized on agarose beads *via* N-hydroxy-succinimide amine coupling chemistry. They first tested if this column was functional by loading hIgG on a 1 ml C3 column. After washing, bound hIgGs were eluted with an acidic buffer pH 2.5. According to the chromatogram in Figure 11 A, C3 is still active once immobilized on agarose beads. However, there were some proteins in fractions corresponding to flow through, indicating that at least a fraction of hIgG was not bound by the column.

According to specificity study by ELISA, the binding of C3 to hIgG2 and to hIgG3 are both weak and difficult to rank (Fig. 5). However, by SPR there is a binding of C3 to hIgG2 but not to hIgG3 (Fig. 7). To check to which extent such weak affinities can be balanced or not by a rebinding effect on a highly dense surface of C3, the specificity of C3 was studied under standard chromatographic conditions. hIgG1, hIgG2, hIgG3 and hIgG4 were separately loaded on the 1 ml C3 column (Fig 11B-11E). This study showed that immobilized C3 was able to fully bind hIgG1, hIgG2 and hIgG4 but not hIgG3. These results suggest that the unbound fraction for hIgG purification (Fig. 11A) could correspond to hIgG3.

Similar experiments with other immunoglobulins showed that C3 is unable to bind to IgG from mouse, rat, sheep, goat, rabbit and hIgA (Fig. 11F-11J, 11L). However, C3 was able to weakly bind to IgG from pig as about 50% of IgG were found in the flow through (Fig. 11K), confirming the weak signal observed by ELISA (Fig. 5). All these result show that C3 has a narrow specificity.

To further test the selectivity of C3 under chromatographic conditions, pure hIgG was mixed either with the soluble fraction of an E. *coli* crude extract or with DMEM medium supplemented with 10% calf serum. Fractions corresponding to flow through and elution steps were analyzed by reducing SDS-PAGE (Fig. 11M, 11N). According to this analysis, hIgG were eluted as pure as the hIgG used to prepare the mix, indicating that C3 is able to discriminate hIgG from numerous exogenous proteins. For comparison, the same samples were also loaded on 1 ml Protein A and Protein G columns. As seen from SDS-PAGE analysis of eluted fractions, the degree of purity reached for hIgG by C3 compared well with those of Protein A and Protein G purification systems (figure 11O-11R).

### Example 6: Importance of residues C24 and N35 in IgG binding

The inventors observed the formation of covalent dimers upon storage of C3 protein when no DTT was present in the buffer. To demonstrate that cysteine 24 was implied in this dimerization, they changed it to a serine (C3-C24S). This mutant protein was indeed not able to form dimers. Since this cysteine was maintained during the selection of IgG binders, it could however have an important role for IgG binding. The affinity of C3-C24S was determined by SPR, and a decrease by a factor of 2.4 was found compared to C3 (230 nM versus 94 nM). This suggests that cysteine 24 is not so critical for the recognition of hIgG.

Upon selection, an aspartate at position 35 in C3 sequence was replaced by an asparagine although position 35 was not encoded as a randomized position in libraries. To understand the importance of this mutation, the asparagine 35 was changed to aspartate in C3-C24S to get the double mutant C3-C24S/N35D. As determined by SPR, this double mutant had an affinity for hIgG decreased by a factor of 135 compare to C3-C24S (31000 nM versus 230 nM). This shows that asparagine 35 has a critical role for the hIgG recognition. Visual inspection of the three dimensional structure of Sac7d shows that aspartate 35 is located on the side of the potential binding area as designed in libraries. Thus, the importance of this position 35 suggests that the binding site for IgG on Sac7d spans this region. Alternatively, asparagine 35 could have a structural and not a functional role (direct interaction with the ligand). In the latter case, this position could be considered as a second sphere position that modulates affinity by a fine positioning of residues that directly interact with the ligand.

### Example 7: Discussion

Previously, the inventors designed a library of Sac7d that allowed isolation of variants, called binders, with affinity and specificity for a given protein target such as PulD (Mouratou et al., 2007). Here they show the successful selection of binders against another chosen ligand: human IgGs. They reasoned that using Fc fragment as a target, binders with recognition properties independent of IgG variable regions could be obtained.

### Selection of IgG binders and their properties

The binding affinities as determined by SPR were all in the nanomolar range for hIgG1: from several dozens to hundreds of nanomolar. Since binders with high affinities are not desirable to design an affinity chromatography system (Graves and Wu, 1974), the selection pressure was maintained at a low level with short washes. Furthermore, six rounds of selection were performed, but four rounds were sufficient to observe a convergence of the sequences since 54 % of them corresponded to C3 variant. This suggests that the library was rich enough with IgG binders to allow a fast enrichment. The confirmed variant C3 was chosen for further characterizations.

Human IgG1 subclass was well recognized. Considering that about 66% of blood circulating IgGs in normal adults are IgG1 (French, 1986), this was somehow expected. The selectivity of C3 was for hIgG1, hIgG4 and to a less extent for hIgG2. No interaction was observed for hIgG3, even when using a test with a high density of binding sites such as an affinity column that allows rebinding events to occur. However, IgG binders were quite specific as they showed no recognition of antibodies from other species than Human and Pig. No selection pressure was imposed to prevent a potential inter species cross-reactivity. There are high sequence identities of Fc regions between the four human subclasses (around 95%). The weak recognition observed for hIgG2 and absence of recognition for hIgG3 probably result from just one or few amino acid differences between sub-classes. This profile of recognition is different from those observed for natural IgG binders such as Protein A and Protein G (Table 1). For IgG1 recognition, C3 compared well with natural IgG binders, especially for particular cases where a hIgG needs to be discriminated from other IgG species.

The high yield of production obtained from *E. coli* flask cultures for several anti-IgG confirms what was observed previously for PulD binders (Mouratou et al., 2007). However, some anti-IgG variants have a low expression yield. This is not surprising considering that more than 21% of Sac7d residues are mutated (14/66) and that there is significant risks that mutations not compatible with folding or with stability to appear. But outputs of selections are sufficiently diverse to find variants with high expression yields such as C3. This variant was shown to be stable under extreme acidic conditions (fig. 10A). Thus, IgG binders kept the favorable biophysical properties of their parent protein Sac7d, which is well expressed in *E. coli* and acidophilic (McCrary et al., 1996).

However C3 was less stable under extreme alkaline conditions. An irreversible denaturation occurred for high pH that is probably due to asparagines and/or glutamine deamidation as monitored by mass spectrometry (data not shown). Sequence of C3 contains five asparagines and one glutamine compared to the wild type Sac7d that contains only one asparagine and no glutamine. Deamidation is an irreversible event that occurs at high pH and is faster if an asparagine is followed by a glycine (Tyler-Cross and Schirch, 1991). In C3, there are two NG combinations. According to figure 9, there is room to improve C3 for alkaline resistance. Indeed, the wild type Sac7d, although described as an acidophilic protein is also quite resistant to high pH. For example at pH 12 no decrease of ellipticity could be observed after overnight incubation.

### Use of IgG binders for immuno-detections

The inventors have shown that C3 covalently labeled with the fluorophore AlexaFluor 680 can be used as a detection reagent for western blot experiments. The results showed that the labeled C3 has an exquisite specificity of recognition as it was able to discriminate hIgG from numerous exogenous proteins. One can imagine using IgG binders genetically fused to a reporter protein such as GFP or PhoA as was showed for PulD binders (Mouratou et al., 2007).

### Use of IgG binders for affinity chromatography

Affinity chromatography is an application particularly drastic for the reagent of capture to be used. Ideally the reagent must be 1) specific of the target to be purified, 2) functional once immobilized in the column, 3) available in large quantities, 4) cheap and easy to produce, 5) stable to acidic pH for elution and 6) resistant to alkaline pH to allow standard cleaning in place procedures to allow reuses of the column. Depending on specific applications other characteristics are desirable, such as resistance to proteases to prevent degradation of the column and also contamination of the purified sample with peptides that could make the preparation incompatible with therapeutic uses, or that will interfere with analysis of the purified antibody (Godfrey et al., 1993).

Getting all these characteristics in one molecule is particularly challenging. The variant C3 has the qualities for five of the six required criteria to use it for affinity chromatography. Indeed, it was shown that once immobilized on agarose beads through standard NHS chemistry, C3 is still specific of the target (no co-purification of proteins from E. *coli* crude extract or supplemented DMEM medium), available in large quantities from a cheap expression system and resistant to the dozens of elutions needed for this work with a buffer at pH 2.5.

The comparison of C3 affinity purifications with Protein A and Protein G, two standard systems for antibodies purification, showed that the C3 system is at least as efficient as them in term of degree of purity reached. Furthermore, it was shown that for all affinity chromatography runs with a C3 column, the Ig found in the flow through and in the elution fractions corresponded to the quantity of Ig injected, indicating that this purification system is quantitative and that no Ig is irreversibly trapped in the C3 column (see SDS-PAGE gels, Fig. 12).

The profile of recognition of C3 for Ig is however different from those of Protein A and Protein G (Table 2). According to this table, C3 profile is much narrower than that of Protein G which recognizes most of IgG. C3 profile is closer than that of Protein A, but still different and narrower. Indeed, C3 is not able to recognize several IgG from species other than man and pig, contrary to Protein A, but recognizes the same human isotypes as Protein A.

**Table 2: Selectivity of C3, Protein A and Protein G. ^{a}: from this study. ^{b}: from "Antibody purification handbook", General Electric Healthcare.**

| IgG | C3^{a} | Protein A^{b} | Protein G^{b} |
|---|---|---|---|
| Human IgG1 | ++++ | ++++ | ++++ |
| Human IgG2 | ++ | ++++ | ++++ |
| Human IgG3 | - | - | ++++ |
| Human IgG4 | +++ | ++++ | ++++ |
| Human IgA | - | variable | - |
| Goat IgG | - | - | ++ |
| Mouse IgG | - | +++ | ++++ |
| Rabbit IgG | - | ++++ | +++ |
| Rat IgG | - | +/- | ++ |
| Sheep IgG | - | +/- | ++ |
| Pig IgG | +/- | +++ | +++ |

To date most of approved human or humanized therapeutic antibodies are mostly IgG1 but also to a less extent IgG2 and IgG4 (Salfeld, 2007). These three isotypes are those recognized by C3. Thus C3 would be adapted to applications where large quantities of therapeutic antibodies need to be purified whatever the isotype commonly used.

Synthetic compounds have been proposed as alternative to natural bacterial binders for Ig purification: peptide mimetics, dyes, amino acids, thiol and triazine based binders (D'Agostino et al., 2008). These systems although interesting in term of costs and stability, often have low selectivity that finally imposes another purification step (Huse et al., 2002; Roque et al., 2007).

### CONCLUSIONS

The results presented in this work further validate the use of Sac7d as a scaffold to obtain binders with affinity and specificity for a given target. Biophysical properties observed for IgG binders were globally favorable with a high resistance to harsh acidic conditions for example. Further work is required to improve C3 stability at high pH in order to allow its use for affinity chromatography applications where the column is reused for different batches and the column is submitted to repetitive standard cleaning in place procedures using NaOH. However, C3 is remarkably well produced in *E*. *coli* without the need of a bioreactor, making its cost of production attractive. Even with its limited resistance to high pH, C3 could be used to design a column for each batch of purification when one wants to strictly avoid cross contamination from batch to batch.

### REFERENCES

Binz, H.K., Amstutz, P., Kohl, A., Stumpp, M.T., Briand, C., Forrer, P., Grutter, M.G. and Pluckthun, A. (2004) High-affinity binders selected from designed ankyrin repeat protein libraries. Nat Biotechnol, 22, 575-582.
Bjorck, L. (1988) Protein L. A novel bacterial cell wall protein with affinity for Ig L chains. J Immunol, 140, 1194-1197.
Bjorck, L. and Kronvall, G. (1984) Purification and some properties of streptococcal protein G, a novel IgG-binding reagent. J Immunol, 133, 969-974.
D'Agostino, B., Bellofiore, P., De Martino, T., Punzo, C., Rivieccio, V. and Verdoliva, A. (2008) Affinity purification of IgG monoclonal antibodies using the D-PAM synthetic ligand: chromatographic comparison with protein A and thermodynamic investigation of the D-PAM/IgG interaction. J Immunol Methods, 333, 126-138.
Fassina, G., Ruvo, M., Palombo, G., Verdoliva, A. and Marino, M. (2001) Novel ligands for the affinity-chromatographic purification of antibodies. J Biochem Biophys Methods, 49, 481-490.
Forsgren, A. and Sjoquist, J. (1966) "Protein A" from S. aureus. I. Pseudo-immune reaction with human gamma-globulin. J Immunol, 97, 822-827.
French, M. (1986) Serum IgG subclasses in normal adults. Monogr Allergy, 19, 100-107.
Godfrey, M.A., Kwasowski, P., Clift, R. and Marks, V. (1993) Assessment of the suitability of commercially available SpA affinity solid phases for the purification of murine monoclonal antibodies at process scale. J Immunol Methods, 160, 97-105.
Graves, D.J. and Wu, Y.T. (1974) On predicting the results of affinity procedures. Methods Enzymol, 34, 140-163.
Huse, K., Bohme, H.J. and Scholz, G.H. (2002) Purification of antibodies by affinity chromatography. J Biochem Biophys Methods, 51, 217-231.
Karlsson, R. and Falt, A. (1997) Experimental design for kinetic analysis of protein-protein interactions with surface plasmon resonance biosensors. J Immunol Methods, 200, 121-133.
Karlsson, R., Katsamba, P.S., Nordin, H., Pol, E. and Myszka, D.G. (2006) Analyzing a kinetic titration series using affinity biosensors. Anal Biochem, 349, 136-147.
Krehenbrink, M., Chami, M., Guilvout, I., Alzari, P.M., Pecorari, F. and Pugsley, A.P. (2008) Artificial binding proteins (Affitins) as probes for conformational changes in secretin PulD. J Mol Biol, 383, 1058-1068.
Li, R., Dowd, V., Stewart, D.J., Burton, S.J. and Lowe, C.R. (1998) Design, synthesis, and application of a protein A mimetic. Nat Biotechnol, 16, 190-195.
McCrary, B.S., Edmondson, S.P. and Shriver, J.W. (1996) Hyperthermophile protein folding thermodynamics: differential scanning calorimetry and chemical denaturation of Sac7d. J Mol Biol, 264, 784-805.
Mouratou, B., Schaeffer, F., Guilvout, I., Tello-Manigne, D., Pugsley, A.P., Alzari, P.M. and Pecorari, F. (2007) Remodeling a DNA-binding protein as a specific in vivo inhibitor of bacterial secretin PulD. Proc Natl Acad Sci USA, 104, 17983-17988.
Ronnmark, J., Gronlund, H., Uhlen, M. and Nygren, P.A. (2002) Human immunoglobulin A (IgA)-specific ligands from combinatorial engineering of protein A. Eur JBiochem, 269, 2647-2655.
Roque, A.C., Silva, C.S. and Taipa, M.A. (2007) Affinity-based methodologies and ligands for antibody purification: advances and perspectives. J Chromatogr A, 1160, 44-55.
Salfeld, J.G. (2007) Isotype selection in antibody engineering. Nat Biotechnol, 25, 1369-1372.
Terpe, K. (2003) Overview of tag protein fusions: from molecular and biochemical fundamentals to commercial systems. Appl Microbiol Biotechnol, 60, 523-533.
Tyler-Cross, R. and Schirch, V. (1991) Effects of amino acid sequence, buffers, and ionic strength on the rate and mechanism of deamidation of asparagine residues in small peptides. J Biol Chem, 266, 22549-22556.

## Claims

1. An immunoglobulin-binding protein of sequence SVKVKF**X₁X₂X₃**GEEKEVDTSKI**RD**V**C**R**Q**GK**N**V**K**F**L**Y**N**DNGK**Y**G**A**G**X₄**V**X₅**EKDAP KELLDMLARAEREKK (SEQ ID No: 1), wherein X₁ to X₅ are, independently from each other, any amino acids, wherein said immunoglobulin-binding protein has an apparent affinity for human IgG so that Kd ≤ 100 µM.

2. An immunoglobulin-binding protein whose sequence is derived from SEQ ID No: 1 by one, two, three or four mutations or deletions in positions selected in the group consisting of positions 1-6, 10-20, 23, 25, 27-28, 30, 32, 34, 36-39, 41, 43, 45 and 47-66, wherein said immunoglobulin-binding protein has an apparent affinity for human IgG so that Kd ≤ 100 µM.

3. The immunoglobulin-binding protein of claim 1 or claim 2, which has an apparent affinity for human IgG so that Kd ≤ 10 µM.

4. The immunoglobulin-binding protein of any of claims 1 to 3, which has an apparent affinity for human IgG so that Kd ≤ 1 µM.

5. The immunoglobulin-binding protein of any of claims 1 to 4, which has an apparent affinity for human IgG so that Kd ≤ 500 nM.

6. The immunoglobulin-binding protein of any of claims 1 to 5, wherein the indicated apparent affinity is for human IgG1 isotype.

7. The immunoglobulin-binding protein of any of claims 1 to 6, wherein **X₁X₂X₃** is selected in the group consisting of LLN, KYK, HSH, LCH, RYL and ARH.

8. The immunoglobulin-binding protein of any of claims 1 to 7, wherein X₄ is N or K and X₅ is D, N or R.

9. The immunoglobulin-binding protein of any of claims 2 to 8, wherein said one, two, three or four mutations or deletions are in positions selected in the group consisting of positions 13, 24, 36, 38, 47, 50 and 56.

10. The immunoglobulin-binding protein of any of claims 1 to 9, which is selected in the group consisting of:

SVKVKFLLNGEEKEVDTSKIRDVCRQGKNVKFLYNDNGKYGAGNVDEKDAPKEL
LDMLARAEREKK (SEQ ID No: 2),
SVKVKFLLNGEEKEVDTSKIRDVCRQGKNVKFLYNDNGKYGAGKVNEKDAPKEL
LDMLARAEREKK (SEQ ID No: 3),
SVKVKFHSHGEEKEVDTSKIRDVCRQGKNVKFLYNDNGKYGAGNVDEKDAPKEL
LDMLARAEREKK (SEQ ID No: 4),
SVKVKFLCHGEEKEVDTSKIRDVCRQGKNVKFLYNDNGKYGAGNVDEKDAPKEL
LDMLARAEREKK (SEQ ID No: 5),
SVKVKFRYLGEEKEVDTSKIRDVCRQGKNVKFLYNDNGKYGAGNVDEKDAPKEL
LDMLARAEREKK (SEQ ID No: 6),

SVKVKFARHGEEKEVDTSKIRDVCRQGKNVKFLYNDNGKYGAGNVREKDAPKEL
LDMLARAEREKK (SEQ ID No: 7),
SVKVKFLLNGEEKEVDTSKIRDVCRQGKNVKFLYNDNGKYGAGNVDEKDVPKEL
LDMLARAEREKK (SEQ ID No: 8),
SVKVKFLLNGEEQEVDTSKIRDVCRQGKNVKFLYNDNGKYGAGNVDEKDAPKEL
LDMLARAEREKK (SEQ ID No: 9)
SVKVKFLLNGEEKEVDTSKIRDVCRQGKNVKFLYNDNGKYGAGNVDKKDAPKEL
LDMLARAEREKK (SEQ ID No: 10)
SVKVKFLLNGEEKEVDTSKIRDVCRQGKNVKFLYNDNDKYGAGNVDEKDAPKEL
LDMLARAEREKK (SEQ ID No: 11)
SVKVKFLLNGEEKEVDTSKIRDVCRQGKNVKFLYNNNGKYGAGNVDEKDAPKEL
LEMLARAEREKK (SEQ ID No: 12).

11. A chimeric protein comprising an immunoglobulin-binding protein according to any of claims 1 to 10, coupled to a second protein moiety.

12. The chimeric protein according to claim 11, which is a fusion protein.

13. The chimeric protein according to claim 11 or claim 12, wherein said second moiety is a reporter protein.

14. The chimeric protein according to claim 11 or claim 12, wherein said second moiety is a binding moiety.

15. Use of an immunoglobulin-binding protein according to any of claims 1 to 10, or of a chimeric protein according to any of claims 11 to 14, for performing a detection assay.

16. Use of an immunoglobulin-binding protein according to any of claims 1 to 10, or of a chimeric protein according to any of claims 11 to 14, for performing a capture assay.


SEQUENCE LISTING

<110>  INSTITUT PASTEUR
       CENTRE NATIONAL DE LA RECHERCHE SCIENTIFIQUE

<120>  IMMUNOGLOBULIN-BINDING PROTEINS DERIVED FROM Sac7d AND USES
       THEREOF

<130>  VMAahF226/174

<160>  15

<170>  PatentIn version 3.3

<210>  1
<211>  66
<212>  PRT
<213>  Artificial

<220>
<223>  antibody-binding protein


<220>
<221>  MISC_FEATURE
<222>  (7)..(9)
<223>  X=any amino acid

<220>
<221>  MISC_FEATURE
<222>  (44)..(44)
<223>  X=any amino acid

<220>
<221>  MISC_FEATURE
<222>  (46)..(46)
<223>  X=any amino acid

<400>  1

Ser Val Lys Val Lys Phe Xaa Xaa Xaa Gly Glu Glu Lys Glu Val Asp
1               5                   10                  15


Thr Ser Lys Ile Arg Asp Val Cys Arg Gln Gly Lys Asn Val Lys Phe
            20                  25                  30


Leu Tyr Asn Asp Asn Gly Lys Tyr Gly Ala Gly Xaa Val Xaa Glu Lys
        35                  40                  45


Asp Ala Pro Lys Glu Leu Leu Asp Met Leu Ala Arg Ala Glu Arg Glu
    50                  55                  60


Lys Lys
65


<210>  2
<211>  66
<212>  PRT
<213>  Artificial

<220>

<223>  antibody-binding protein

<400>  2

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210>  3
<211>  66
<212>  PRT
<213>  Artificial

<220>
<223>  antibody-binding protein

<400>  3

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Lys Val Asn Glu Lys
        35                  40                  45

Asp Ala Pro Lys Glu Leu Leu Asp Met Leu Ala Arg Ala Glu Arg Glu
    50                  55                  60

Lys Lys
65

<210>  4
<211>  66
<212>  PRT
<213>  Artificial

<220>
<223>  antibody-binding protein

<400>  4

Ser Val Lys Val Lys Phe His Ser His Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210>  5
<211>  66
<212>  PRT
<213>  Artificial

<220>
<223>  antibody-binding protein

<400>  5

Ser Val Lys Val Lys Phe Leu Cys His Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210>  6
<211>  66
<212>  PRT
<213>  Artificial

<220>
<223>  antibody-binding protein

<400>  6

Ser Val Lys Val Lys Phe Arg Tyr Leu Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210>  7
<211>  66
<212>  PRT
<213>  Artificial

<220>
<223>  antibody-binding protein

<400>  7

Ser Val Lys Val Lys Phe Ala Arg His Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Arg Glu Lys
        35                  40                  45

Asp Ala Pro Lys Glu Leu Leu Asp Met Leu Ala Arg Ala Glu Arg Glu
    50                  55                  60

Lys Lys
65

<210>  8
<211>  66
<212>  PRT
<213>  Artificial

<220>
<223>  antibody-binding protein

<400>  8

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Val Pro Lys Glu Leu Leu Asp Met Leu Ala Arg Ala Glu Arg Glu
    50                  55                  60

Lys Lys
65

<210> 9
<211> 66
<212> PRT
<213> Artificial

<220>
<223> antibody-binding protein

<400> 9

Ser Val Lys Val Lys Phe Leu Leu Asn Gly Glu Glu Gln Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210> 10
<211> 66
<212> PRT
<213> Artificial

<220>
<223> antibody-binding protein

<400> 10

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Lys Lys
        35                  40                  45

Asp Ala Pro Lys Glu Leu Leu Asp Met Leu Ala Arg Ala Glu Arg Glu
    50                  55                  60

Lys Lys
65

<210>  11
<211>  66
<212>  PRT
<213>  Artificial

<220>
<223>  antibody-binding protein

<400>  11

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Asp Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210>  12
<211>  66
<212>  PRT
<213>  Artificial

<220>
<223>  antibody-binding protein

<400>  12

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asn Asn Gly Lys Tyr Gly Ala Gly Asn Val Asp Glu Lys
        35                  40                  45

Asp Ala Pro Lys Glu Leu Leu Glu Met Leu Ala Arg Ala Glu Arg Glu
    50                  55                  60

Lys Lys
65

<210>  13
<211>  41
<212>  DNA
<213>  Artificial

<220>

<223>  oligonucléotide

<400>  13
gcagccaagc ttaattaatg actgagcttg gactcctgtt g                41


<210>  14
<211>  20
<212>  DNA
<213>  Artificial

<220>
<223>  oligonucléotide

<400>  14
cgccaagcta gcttggattc                                        20


<210>  15
<211>  32
<212>  DNA
<213>  Artificial

<220>
<223>  primer

<400>  15
tcggcccccg aggccatata aagctttttc tc                          32